# EUROPEAN PATENT SPECIFICATION

(11) **EP 1 560 923 B1**
(45) Date of publication and mention of the grant of the patent: **25.07.2007**
(21) Application number: 03750698.7
(22) Date of filing: 08.10.2003
(51) Int. Cl.: C12Q 1/48

(54) **ASSAYS FOR C-MANNOSYLTRANSFERASE**
ASSAYS FÜR C-MANNOSYLTRANSFERASE
CRIBLAGES RELATIFS A LA C-MANNOSYLTRANSFERASE

(30) Priority: 09.10.2002 GB 0223449
(43) Date of publication of application: 10.08.2005
(73) Proprietor: Novartis Forschungsstiftung, Zweigniederlassung Friedrich Miescher Institute for Biomedical Research, 4058 Basel (CH)
(72) Inventor: HOFSTEENGE, Jan, CH-4153 Reinach (CH); KEUSCH, Jeremy, CH-4054 Basel (CH)
(86) International application number: PCT/EP2003/011139
(87) International publication number: WO 2004/033712

(56) References cited:
- KEUSCH JEREMY J ET AL: "C-mannosylation of cell surface glycoproteins." GLYCOBIOLOGY, vol. 12, no. 10, October 2002 (2002-10), page 674, XP009036292 & 7TH ANNUAL CONFERENCE OF THE SOCIETY FOR GLYCOBIOLOGY; BOSTON, MA, USA; NOVEMBER 09-12, 2002 ISSN: 0959-6658
- DOUCEY M-A ET AL: "PROTEIN C-MANNOSYLATION IS ENZYME-CATALYSED AND USES DOLICHYL-PHOSPHATE-MANNOSE AS A PRECURSOR" MOLECULAR BIOLOGY OF THE CELL, BETHESDA, MD, US, vol. 9, no. 2, February 1998 (1998-02), pages 291-300, XP008001070 ISSN: 1059-1524 cited in the application
- KRIEG ET AL: "C-mannosylation of human RNase 2 is an intracellular process performed by a variety of cultured cells" JOURNAL OF BIOLOGICAL CHEMISTRY, AMERICAN SOCIETY OF BIOLOGICAL CHEMISTS, BALTIMORE, MD, US, vol. 272, no. 42, 17 October 1997 (1997-10-17), pages 26687-26692, XP002192199 ISSN: 0021-9258 cited in the application

### SEQUENCE LISTING

<110> Novartis Forschungsstiftung, zweigniederlassung Friedrich Miescher Institute for Biomedical Research
   hofsteenge, jan
   keusch, jeremy
<120> Assays for c-Mannosyltransferase
<130> 1-32709A
<150> GB0223449.0
<151> 2002-10-09
<160> 9
<170> PatentIn version 3.1
<210> 1
<211> 6
<212> PRT
<213> Artificial sequence
<220>
<223> synthetic peptide
<400> 1
   Ala Trp Ala Gln Trp Ala
   1               5
<210> 2
<211> 117
<212> DNA
<213> artificial sequence
<220>
<223> synthetic oligonucleotide
<400> 2
   acagaagctt ccaccaatgg ttccaaaact gttcacttcc caaatttgtc tgcttcttct
   60
   gttggggctt ctggctgtgg agggctcact ccatgtctcc cctatactag gttattg
   117
<210> 3
<211> 55
<212> DNA
<213> artificial sequence
<220>
<223> synthetic oligonucleotide
<400> 3
   atatgaattc tcagtcagtc aagcccattt agcccaagcg gatccacgcg gaacc
   55
<210> 4
<211> 50
<212> DNA
<213> artificial sequence
<220>
<223> synthetic oligonucleotide
<400> 4
   ggggtaccag atctaagctt gccaccatgg ccaagggctt ctatatttcc
   50
<210> 5
<211> 50
<212> DNA
<213> artificial sequence
<220>
<223> synthetic oligonucleotide
<400> 5
   gactagtacc ctgaaaatac aaattctcac tttggtccaa ggtggtggcc
   50
<210> 6
<211> 28
<212> DNA
<213> artificial sequence
<220>
<223> synthetic oligonucleotide
<400> 6
   gactagttcc cctatactag gttattgg
   28
<210> 7
<211> 42
<212> DNA
<213> artificial sequence
<220>
<223> synthetic oligonucleotide
<400> 7
   gctctagaga attcctatca agcccactga gcccaagcgg at
   42
<210> 8
<211> 924
<212> DNA
<213> artificial sequence
<220>
<223> engineered sequence
<220>
<221> CDS
<222> (1)..(924)
<223>
<400> 8
   atg gcc aag ggc ttc tat att tcc aag tcc ctg ggc atc ctg ggg atc
   48
   Met Ala Lys Gly Phe Tyr Ile Ser Lys Ser Leu Gly Ile Leu Gly Ile
   1 5 10 15
   ctc ctg ggc gtg gca gcc gtg tgc aca atc atc gca ctg tca gtg gtg
   96
   Leu Leu Gly Val Ala Ala Val Cys Thr Ile Ile Ala Leu Ser Val Val
   20 25 30
   tac tcc cag gag aag aac aag aac gcc aac agc tcc ccc gtg gcc tcc
   144
   Tyr Ser Gln Glu Lys Asn Lys Asn Ala Asn Ser Ser Pro Val Ala Ser
   35 40 45
   acc acc ccg tcc gcc tca gcc acc acc aac ccc gcc tcg gcc acc acc
   192
   Thr Thr Pro Ser Ala Ser Ala Thr Thr Asn Pro Ala Ser Ala Thr Thr
   50 55 60
   ttg gac caa agt gag aat ttg tat ttt cag ggt act agt tcc cct ata
   240
   Leu Asp Gln Ser Glu Asn Leu Tyr Phe Gln Gly Thr Ser Ser Pro Ile
   65 70 75 80
   cta ggt tat tgg aaa att aag ggc ctt gtg caa ccc act cga ctt ctt
   288
   Leu Gly Tyr Trp Lys Ile Lys Gly Leu Val Gln Pro Thr Arg Leu Leu
   85 90 95
   ttg gaa tat ctt gaa gaa aaa tat gaa gag cat ttg tat gag cgc gat
   336
   Leu Glu Tyr Leu Glu Glu Lys Tyr Glu Glu His Leu Tyr Glu Arg Asp
   100 105 110
   gaa ggt gat aaa tgg cga aac aaa aag ttt gaa ttg ggt ttg gag ttt
   384
   Glu Gly Asp Lys Trp Arg Asn Lys Lys Phe Glu Leu Gly Leu Glu Phe
   115 120 125
   ccc aat ctt cct tat tat att gat ggt gat gtt aaa tta aca cag tct
   432
   Pro Asn Leu Pro Tyr Tyr Ile Asp Gly Asp Val Lys Leu Thr Gln Ser
   130 135 140
   atg gcc atc ata cgt tat ata gct gac aag cac aac atg ttg ggt ggt
   480
   Met Ala Ile Ile Arg Tyr Ile Ala Asp Lys His Asn Met Leu Gly Gly
   145 150 155 160
   tgt cca aaa gag cgt gca gag att tca atg ctt gaa gga gcg gtt ttg
   528
   Cys Pro Lys Glu Arg Ala Glu Ile Ser Met Leu Glu Gly Ala Val Leu
   165 170 175
   gat att aga tac ggt gtt tcg aga att gca tat agt aaa gac ttt gaa
   576
   Asp Ile Arg Tyr Gly Val Ser Arg Ile Ala Tyr Ser Lys Asp Phe Glu
   180 185 190
   act ctc aaa gtt gat ttt ctt agc aag cta cct gaa atg ctg aaa atg
   624
   Thr Leu Lys Val Asp Phe Leu Ser Lys Leu Pro Glu Met Leu Lys Met
   195 200 205
   ttc gaa gat cgt tta tgt cat aaa aca tat tta aat ggt gat cat gta
   672
   Phe Glu Asp Arg Leu Cys His Lys Thr Tyr Leu Asn Gly Asp His Val
   210 215 220
   acc cat cct gac ttc atg ttg tat gac gct ctt gat gtt gtt tta tac
   720
   Thr His Pro Asp Phe Met Leu Tyr Asp Ala Leu Asp Val Val Leu Tyr
   225 230 235 240
   atg gac cca atg tgc ctg gat gcg ttc cca aaa tta gtt tgt ttt aaa
   768
   Met Asp Pro Met Cys Leu Asp Ala Phe Pro Lys Leu Val Cys Phe Lys
   245 250 255
   aaa cgt att gaa gct atc cca caa att gat aag tac ttg aaa tcc agc
   816
   Lys Arg Ile Glu Ala Ile Pro Gln Ile Asp Lys Tyr Leu Lys Ser Ser
   260 265 270
   aag tat ata gca tgg cct ttg cag ggc tgg caa gcc acg ttt ggt ggt
   864
   Lys Tyr Ile Ala Trp Pro Leu Gln Gly Trp Gln Ala Thr Phe Gly Gly
   275 280 285
   ggc gac cat cct cca aaa tcg gat ctg gtt ccg cgt gga tcc gct tgg
   912
   Gly Asp His Pro Pro Lys Ser Asp Leu Val Pro Arg Gly Ser Ala Trp
   290 295 300
   gct cag tgg gct
   924
   Ala Gln Trp Ala
   305
<210> 9
<211> 308
<212> PRT
<213> artificial sequence
<220>.
<223> engineered sequence
<400> 9
   Met Ala Lys Gly Phe Tyr Ile Ser Lys Ser Leu Gly Ile Leu Gly Ile
   1               5                   10                  15
   Leu Leu Gly Val Ala Ala Val Cys Thr Ile Ile Ala Leu Ser Val Val
               20                  25                  30
   Tyr Ser Gln Glu Lys Asn Lys Asn Ala Asn Ser Ser Pro Val Ala Ser
           35                  40                  45
   Thr Thr Pro Ser Ala Ser Ala Thr Thr Asn Pro Ala Ser Ala Thr Thr
       50                  55                  60
   Leu Asp Gln Ser Glu Asn Leu Tyr Phe Gln Gly Thr Ser Ser Pro Ile
   65                  70                  75                  80
   Leu Gly Tyr Trp Lys Ile Lys Gly Leu Val Gln Pro Thr Arg Leu Leu
                   85                  90                  95
   Leu Glu Tyr Leu Glu Glu Lys Tyr Glu Glu His Leu Tyr Glu Arg Asp
               100                 105                 110
   Glu Gly Asp Lys Trp Arg Asn Lys Lys Phe Glu Leu Gly Leu Glu Phe
   115 120 125
   Pro Asn Leu Pro Tyr Tyr Ile Asp Gly Asp Val Lys Leu Thr Gln Ser
   130 135 140
   Met Ala Ile Ile Arg Tyr Ile Ala Asp Lys His Asn Met Leu Gly Gly
   145 150 155 160
   Cys Pro Lys Glu Arg Ala Glu Ile Ser Met Leu Glu Gly Ala Val Leu
   165 170 175
   Asp Ile Arg Tyr Gly Val Ser Arg Ile Ala Tyr Ser Lys Asp Phe Glu
   180 185 190
   Thr Leu Lys Val Asp Phe Leu Ser Lys Leu Pro Glu Met Leu Lys Met
   195 200 205
   Phe Glu Asp Arg Leu Cys His Lys Thr Tyr Leu Asn Gly Asp His Val
   210 215 220
   Thr His Pro Asp Phe Met Leu Tyr Asp Ala Leu Asp Val Val Leu Tyr
   225 230 235 240
   Met Asp Pro Met Cys Leu Asp Ala Phe Pro Lys Leu Val Cys Phe Lys
   245 250 255
   Lys Arg Ile Glu Ala Ile Pro Gln Ile Asp Lys Tyr Leu Lys Ser Ser
   260 265 270
   Lys Tyr Ile Ala Trp Pro Leu Gln Gly Trp Gln Ala Thr Phe Gly Gly
   275 280 285
   Gly Asp His Pro Pro Lys Ser Asp Leu Val Pro Arg Gly Ser Ala Trp
   290 295 300
   Ala Gln Trp Ala
   305

## Description

The current invention relates to protein modifications, in particular to glycosylated proteins and to the enzyme that catalyzes a particular form of glycosylation, C-mannosylation.

### BACKGROUND OF THE INVENTION

Glycosylation is one of the most abundant and widespread modifications of proteins and is increasingly being recognized to play an important role in a variety of biological processes. Recent examples are the modulation of Notch signaling by O-fucosylation (Moloney, D.J. et al., 2000, Nature, 406: 369-375; Brückner, K. et al., 2000, Nature, 406: 411-415), and the function of glucosylation in quality control of protein folding (Ellgaard, L. & Helenius, A., 2001, Curr. Opin. Cell Biol., 13: 431-437). As a consequence, deficiencies in glycoconjugate metabolism are now known to be associated with various pathological situations in humans. For example, Leucocyte Adhesion Deficiency II was shown to be caused by a molecular defect in GDP-fucose transport (Luhn, K. et al., 2001, Nat. Genet., 28: 69-72).

The best-studied forms of protein glycosylation, N- and O-glycosylation, have been known for approximately forty years. In these cases glycans are attached to the protein via an amide or hydroxyl group of an amino acid side chain, respectively. In vitro assays for glycosylases have been described in the literature (see EP 0272603, EP1 260 591, Hendershot et al., 2002, Analytical Biochemistry 307: 273-279).

Hofsteenge and collaborators reported a new protein-carbohydrate linkage, comprising the attachment of an alpha-mannopyranosyl residue to the C-2 atom of tryptophan in human RNase 2 (Hofsteenge, J. et al., 1994, Biochemistry, 33: 13524-13530; de Beer, T. et al., 1995, Biochemistry, 34: 11785-11789). This glycoconjugate does not contain the typical N- or O-glycosidic linkage but rather a C-C bond. The modification reaction is catalyzed by a microsomal C-mannosyltransferase (CMT), which uses dolichyl-phosphate-mannose (DPM) as the sugar donor and recognizes the sequence Trp-x-x-Trp (WXXW) in the protein acceptor (Doucey, M.-A. et al., 1998, Mol. Biol. Cell, 9: 291-300; Krieg, J. et al., 1998, Mol. Biol. Cell, 9: 301-309).

Presently, 49 C-mannosylated tryptophans have been identified, derived from 11 different proteins that perform a variety of functions (see Furmanek, A. & Hofsteenge, J., 2000, Acta Biochim. Pol., 47: 781-789 for a review). Amongst these, human properdin takes a special position, because 15 of its 20 tryptophans are C-mannosylated (Hartmann, S. & Hofsteenge, J., 2000, J. Biol. Chem., 275: 28569-28574). Properdin, a 50-kDa protein present in plasma, is the positive regulator of the complement system, a first line of host defense against microbes. Its three activation pathways are composed of proteolytic cascades, which converge at the step that leads to the conversion of C3 into C3b. The rapid inactivation of the heterodimeric C3 convertase of the alternative pathway, C3bBb, is prevented by the formation of a complex with properdin. Because the produced C3b is involved in the formation of more C3b, a positive feedback loop exists that is stabilized by properdin. Subsequent reactions then lead to the formation of the membrane attack complex (MAC), the actual lytic component of the complement system. This complex consists of six different subunits, five of which are also C-mannosylated on multiple tryptophan residues (Hofsteenge, J. et al., 1999, J. Biol. Chem., 274: 32786-32794).

An *in vitro* CMT assay has been described using radioactively labelled DPM, liver microsomes and synthetic peptides containing a CMT recognition site (WXXW). After extraction of the reaction products with chloroform/methanol, the C-mannosylated peptide is detected in the aqueous phase through the presence of the radioactive label (Doucey, M.-A. et al., 1998). The assay although useful is limited in its application because of technical problems that are experienced, such as with the stability of the peptide, both during the assay and during subsequent isolation, with the delivery of the peptide to the correct cellular compartment, with the isolation of minute amounts of modified material for analysis and with variability of results from cell to cell or cell-type to cell-type, leading generally to problems with reproducibility of the assay. There remains therefore a need for assays, in particular cell-based assays, amenable to high throughput formats to allow the identification of CMT as well as modulators of its activity and this invention meets that need.

### SUMMARY OF THE INVENTION

The present invention provides methods for assaying for C-mannosyltransferase (CMT) activity, comprising the steps of: i) providing CMT and a CMT substrate, preferably in a cell (e.g., as a fusion protein with a transmembrane domain), ii) providing conditions conducive to forming a C-mannosylated CMT substrate by action of the CMT on the CMT substrate; iii) immobilizing the C-mannosylated CMT substrate (e.g., through expression as a cell-surfaced bound protein) and (iv) detecting the C-mannosylated CMT substrate. The methods are particularly useful for screening for agents that modulate CMT activity.

Thus, in another aspect of the invention, methods of identifying agents effective in modulating C-mannosyltransferase (CMT) are provided. The methods comprise the steps of: i) contacting a cell comprising CMT with an agent; ii) detecting CMT activity; and iii) determining the agent-induced modulation in the CMT activity relative to when said agent is absent. Potential inhibitors of CMT activity as well as activators can be identified using these methods.

In a preferred embodiment, the screening methods of the invention are cell-based methods where a cell surface bound CMT substrate is expressed in the cell and C-mannosylation of the substrate is detected at the cell surface, for example, released from the cell surface after cleavage with a protease. In this aspect of the invention, the C-mannosylated substrate will typically be presented as a fusion protein. The C-mannosylated substrate can be detected using a variety of means, such as with antibodies, detectable labels or tags.

To ensure that the modulators do not have undesired effects by modulating DPM synthesis, in the more preferred embodiments, a detectable marker, such as a GPI anchored protein (e.g., CD59) indicating unaffected DPM synthesis is detected.

Agents identified by the screening methods are also provided.

Such methods can also be used to identify the elusive CMT gene(s) using antisense oligonucleotides or siRNAs. A loss of C-mannosylated substrate in the presence of such nucleic acids indicates the presence of inhibitory sequences specific for CMT, which can then be used to search databases and identify the CMT gene(s).

### DETAILED DESCRIPTION OF THE INVENTION

### CMT Activity Assays

The identification of the C-mannosyltransferase (CMT) remains elusive even though substantial efforts have been invested in its purification. The present invention provides a highly sensitive assay to detect C-mannosyltransferase activity, which will aid in the identification and characterization of CMT and also provides means to screen for agents that modulate the enzymatic activity.

In its broadest aspect, the present invention provides methods for assaying for C-mannosyltransferase (CMT) activity, comprising the steps of: i) providing CMT and a CMT substrate, ii) providing conditions conducive to forming a C-mannosylated CMT substrate by action of the CMT on the CMT substrate; iii) immobilizing the C-mannosylated CMT substrate and (iv) detecting the C-mannosylated CMT substrate.

The expression of CMT activity in a variety of cells has been studied and discussed in the scientific literature, including cells of insect, nematodes, amphibians, birds and mammals. Any of these cell and tissue sources or others yet to be discovered may be used as a source of CMT. For some purposes, the CMT will often be obtained from a cell line, such as HEK 293, COS7, CHO or NIH3T3 cells.

In one embodiment of the invention, CMT activity is assayed in vitro after the preparation of a cell extract. Methods for the preparation of cell extracts are known in the art (for example, see Scopes, 1987, Protein Purification: Principles and Practice, Second Edition, Springer-Verlag, N.Y.). Liver microsomes can also be used as the source of CMT as described previously (Doucey, M.-A. et al., 1998). Alternatively, a cell extract can also be prepared merely by lysing a cell sample to release CMT without further sample preparation.

Regardless of the origin of the CMT, the CMT sample (or an aliquot thereof) is assayed in a reaction mixture comprising a CMT substrate under conditions allowing C-mannosylation of the substrate. For, in vitro assays, this may mean incubating the reaction mixture in a buffer at physiological pH and at 37°C. Preferably, the reaction takes place in a cell comprising the CMT substrate and CMT as is further described below. The particular CMT substrate chosen in each case may vary depending on the type or origin of the CMT activity for which one is testing, or the type of detection method employed but will typically be a peptide or polypeptide comprising the consensus sequence WXXW.

The CMT substrate is designed to allow immobilization of the C-mannosylated CMT substrate to a solid phase (which includes a cell surface) or solid matrices. The CMT substrate is immobilized on a solid support in a manner such that C-mannosylation can be detected if the enzyme CMT is present in the sample (or cell). Immobilization can be achieved by any manner provided that detection is not impaired and thus, an oriented attachment of CMT substrate is typically required. Such oriented attachment can be achieved by various methods known in the art, including specific chemical reaction of the terminal end of the protein to reactive groups on the support, the attachment of a tag to the terminal end of the protein which can be captured by a receptor that specifically binds the tag (e.g., GST-antibody, biotin-streptavidin, biotin-avidin, antibody-epitope interactions could be used as receptor-tag pairs) or the C-mannosylation site can be used for immobilization using specific antibodies or chemical reactions, for example.

The solid support can be a multiwell plate, a gel, a matrix, a bead, a membrane or a tube, for example, and may be made of various materials, including without limitation, cellulose, agarose, dextran, polycarbonate, nylon, polyethylene, polycarbonates, and glass. Polystyrene beads of about 1 micron to about 5 millimeters in diameter; plates or the wells of a microtiter plate such as those made from polystyrene or polyvinylchloride; glass beads; magnetic particles; polysaccharides; are examples of surfaces that can be coated, e.g., with an antibody. The antibody is typically affixed to the solid matrix by adsorption from an aqueous medium although other modes of affixation, as is well known to those of ordinary skill in the art, can also be used. The amount of antibody coated on the solid surface can be varied by adjusting the concentration of antibody in the contacting solution. In preferred embodiments, the solid support can be a membrane or surface, such as provided by a liposome or a cell.

Thus, in preferred embodiments of the invention, the assay is carried out as a cell-based assay, for which it is advantageous to include a transmembrane domain in a fusion protein comprising the CMT substrate for expression on the surface of a cell. The transmembrane domain expressed as part of the fusion protein can thus be envisioned as a "tag" immobilizing the fusion protein to the cell surface. A transmembrane domain comprises hydrophobic regions that can be identified as such by computer-aided hydropathy plots and allows anchorage of the peptide to the cell's membrane. The transmembrane domain can be of any origin and is exemplified below using the aminopeptidase N transmembrane domain. Example 1 below illustrates the invention using a construct expressing a CMT substrate that is C-mannosylated in the CMT activity assay resulting in a membrane bound CMT substrate.

The construct encodes a fusion protein having an N-terminal domain comprising the cytoplasmic tail, transmembrane and stalk regions of human aminopeptidase N (APN) followed in N-terminal to C-terminal order, by a tobacco etch virus (TEV) protease site, glutathione S-transferase, a thrombin cleavage site and the CMT substrate, AWAQWA (SEQ ID No:1), or multiple copies thereof, expressed under the control of the CMV promoter. In some embodiments, the presence of multiple copies of the CMT substrate is particularly desirable, for example, when a reporter is immobilized and detected, or when it is desired to saturate the CMT with substrate so that activators can be found. As is apparent to one of ordinary skill in the art, variations in the design of the fusion protein are within the scope of the invention. In some embodiments, where anchorage to a cell membrane is not desired, it is also possible to use a polypeptide expression cassette additionally containing a signal sequence that causes the protein to be secreted into the medium.

In another embodiment, the CMT substrate can be linked to a reporter molecule. The reporter molecule (i.e., a signal generating molecule) can be any molecule capable of providing a detectable change. Such reporter molecules include fluorescent moieties (e.g., fluorescent proteins or chemical fluorescent labels), radioactive moieties, phosphorescent moieties, antigens, reporter enzymes and the like. Preferably, the reporter molecule is a reporter enzyme whose activity brings about a detectable change. Such reporter enzymes include, but are not limited to, the following: beta-galactosidase, glucosidases, chloramphenicol acetyltransferase (CAT), glucoronidases, luciferase, peroxidases, phosphatases, oxidoreductases, dehydrogenases, transferases, isomerases, kinases, reductases, deaminases, catalases and urease.

In selecting a reporter molecule to be used in the methods of the inventions, it is imperative that the reporter molecule is not subject to inactivation by any agent in the sample, including inactivation by any protease activity present in a sample. The selection of an appropriate reporter molecule will be readily apparent to those skilled in the art.

Linkage of the tags, reporters, or polypeptides or peptides with other desired characteristics to the CMT substrate can be achieved by any means known in the art (or means yet to be discovered) provided that (i) the CMT substrate can be recognized by the CMT and (ii) oriented immobilization is not impaired. Thus, linkage can be achieved by chemical reaction, by incorporation of a tag (e.g., biotin) during synthesis of the fusion protein, or by contiguous synthesis, such as by using recombinant methods, as is well known in the art, e.g., allowing display of the membrane anchored C-mannosylated CMT substrate extracellularly.

Fusion proteins will most typically be produced by recombinant means, where the expression construct can be introduced into the cells of interest using methods known in the art, for example, by passive internalization, by microporation using a detergent or Staphylococcus alpha toxins, by employing liposomes (e.g., LipofectAmine.TM., Lipofectin.TM., LipofectAce.TM. available commercially), by calcium phosphate mediated internalization, using biolistics, or by electroporation. After the target DNA is internalized by the cell, the sample is incubated to allow synthesis of the substrate and C-mannosylation thereof by CMT.

Therefore, after incubation, the C-mannosylated substrate is either recovered in a cell extract, in the medium (where the substrate is a secreted form) or on the cell's surface. Detection of the C-mannosylated substrate will be governed to some extent by the method used to immobilize the substrate to the solid support. For example, if the substrate is immobilized to a solid matrix, detection using a reporter molecule is only possible if immobilization is via the C-mannosylated moiety (for example, using an antibody specific for C-mannosylated substrate). Immobilization through moieties other than the C-mannosylation would require detection of the C-mannosylated residue, for example using an antibody specific for C-mannosylated substrate. Such an antibody would not recognize the non-C-mannosylated form.

C-mannosylated substrates exhibited at the cell surface can be recognized using specific antibodies, e.g., specific labelled antibodies and identified using FACS analysis, as is described in Example 1 below. Alternatively, if the fusion protein comprising the CMT substrate further comprises one or more protease cleavage sites flanking the CMT substrate, the CMT substrate can be isolated and C-mannosylation identified by mass spectrometry, as is described below in Example 2. Thus, it will be apparent to one of ordinary skill in the art that numerous variations exist for detecting C-mannosylation of a CMT substrate, for example, using an antibody or labelled moiety, such as the use of ³H - mannose present in the buffer or cell. In addition, it will be apparent that the assays are easily amenable to high through-put technologies using robotics and automated processes. The methods are particularly useful for screening for agents that modulate CMT activity.

Screening Assays

In another aspect of the invention, methods of identifying agents effective in modulating C-mannosyltransferase (CMT) are provided. The methods comprise the steps of: i) contacting CMT (or a functional equivalent thereof) in a cell with an agent; ii) detecting CMT activity; and iii) determining the agent-induced modulation in the CMT activity relative to when said agent is absent. Potential inhibitors of CMT activity as well as activators can be identified using these methods. Thus, activators and inhibitors are referred to collectively herein as modulators and preferably influence CMT activity directly (i.e., do not inhibit the DPM pathway).

Exemplary functional equivalents or derivatives of CMT include molecules where CMT is covalently modified by substitution, chemical, enzymatic, or other appropriate means with a moiety other than a naturally occurring amino acid. Derivatives that retain common structural features can be fragments of CMT, in particular fragments maintaining catalytic activity. Preferably, fragments will be at least 50, at least 100 or more amino acids in length. Derivatives of CMT also comprise mutants thereof, which may contain amino acid deletions, additions or substitutions, subject to the requirement to maintain at least one feature characteristic of CMT, preferably catalytic activity. Thus, conservative amino acid substitutions may be made substantially without altering the nature of CMT, as may truncations. Deletions and substitutions may moreover be made to the fragments of CMT used in the screening methods of the invention, in particular those enhancing CMT catalytic activity or providing some other desirable property.

Any of the CMT activity assays described above can be used in the screening assays of the invention. Agents modulating expression of a CMT gene as well as modulating the enzymatic activity will be identified using the assays of the inventions. In a preferred embodiment, the screening methods of the invention are cell-based methods where a cell surface bound CMT substrate is expressed in the cell and C-mannosylation of the substrate is detected at the cell surface or released from the cell surface after cleavage with a protease, for example. In this aspect of the invention, the C-mannosylated substrate will typically be presented as a fusion protein. The C-mannosylated substrate can be detected using a variety of means, such as with antibodies, detectable labels or tags.

To ensure that the modulators do not have undesired effects by modulating dolichyl-phosphate-mannose (DPM) synthesis, in the more preferred embodiments, a detectable marker, such as a glycosylphosphatidylinositol (GPI)-anchored protein (e.g., CD59) indicating unaffected DPM synthesis is detected. CD59 is a 103-amino acid protein present on the surface of a variety of cells. It is anchored to the membrane by means of a GPI anchor. The protein inhibits the formation of the membrane attack complex of the complement system, by associating with C9, preventing its incorporation into C5b-8. This protects cells from complement-mediated lysis (Morgan, B.P. & Harris, C.L., 1999, Complement regulatory proteins, Academic Press, San Diego). However, in this aspect of the invention, CD59 merely serves its function as a marker for DPM synthesis. Other suitable markers will be apparent to those of ordinary skill in the art. If CD59 synthesis (or similar GPI anchor) were decreased, the methods of the invention would thereby indicate the presence of a modulator of GPI anchor synthesizing enzymes, which also provides useful information as lack of GPI is often associated with serious disease. Thus, the methods of the invention can also be used to identify modulators of GPI anchor synthesizing enzymes.

The screening system is preferably used to screen agents that may be present in small molecule libraries, peptide libraries, phage display libraries or natural product libraries. Agent may be inorganic or organic, for example, an antibiotic or antibody. For ease of administration, the agent is preferably a small molecule.

The agent may also be a nucleic acid, such as an antisense oligonucleotide or siRNA. Such agents are particularly useful in identifying the coding sequence of CMT, as a decrease in C-mannosylated substrate would be indicate that the nucleic acid used inhibits CMT expression and is a likely candidate for identifying the CMT coding sequence. A search of nucleic acid databases will identify sequences comprising one or more of the candidates (or their complementary strands), thereby aiding in the discovery of genes of functional importance in C-mannosylation of CMT substrates.

Kits useful for screening agents may also be prepared in accordance with the invention, and will comprise essentially CMT or a functional equivalent thereof useful for screening, and instructions. Typically the CMT will be provided together with one or more of a CMT substrate (preferably the membrane-bound fusion protein described herein), a means for detecting CMT activity and at least one agent (putative agent).

CMT for use in kits according to the invention may be provided in the form of a protein, for example in solution, suspension or lyophilised, or in the form of a nucleic acid sequence permitting the production of CMT or a functional equivalent thereof in an expression system, optionally in situ.

Also provided are methods of identifying agents effective in modulating C-mannosyltransferase (CMT), said method comprising the steps of: (a) providing a non-human wildtype or genetically engineered animal comprising a CMT gene; (b) administering an agent to the non-human animal; and (c) determining whether CMT activity is affected relative to when said agent is absent.

Agents may be identified by screening using the techniques described hereinbefore, and prepared by extraction from natural sources according to established procedures, or by synthesis, especially in the case of low molecular weight chemical agents. Proteinaceous agents may be prepared by expression in recombinant expression systems, for example a baculovirus system, or in a bacterial system. Proteinaceous agents are mainly useful for research into the function of CMT, although they may have a therapeutic application.

Low molecular weight agents, on the other hand, are preferably produced by chemical synthesis according to established procedures. They are primarily indicated as therapeutic agents. Low molecular weight agents and organic agents in general may be useful as agents for use in the treatment of diseases or conditions dependent on CMT activity. Modulators of CMT activity are useful as research tools as well as in various clinical settings and thus, can be used whenever it is desirable to modulate CMT activity. For example, various components of the complement system have been shown to be C-mannosylated and CMT inhibitors may be used to manipulate the complement system in a variety of pathological or therapeutic situations. For instance, complement activation has been implicated in graft rejection after organ transplantation (Bos, I.G., et al., 2002, Transfus. Med. Rev., 16: 251-264), or in reperfusion injury of ischemic myocardium (Monsinjon, T. et al., 2001, Fundam. Clin. Pharmacol., 15: 293-306). Such an inhibition may be accomplished by inactivating essential components of complement like properdin and MAC, which have been shown to be heavily C-mannosylated.

Similarly, C-mannosylation of extracellular matrix proteins (thrombospondin-1 and -2), axonal guidance proteins, F-spondin, M-spondin, SCO-spondin, semaphorins F and G, RNase 2, proteins involved in angiogenesis (e.g., brain-specific angiogenin inhibitors BAI-1, -2 and -3), metalloproteases (ADAMTS), aggrecanase, GON-1, procollagen I N-proteinase, lacunin, TRAP, and cytokine receptors (e.g., receptors for erythropoietin, growth hormone, prolactin, Interleukin-2, -3, -4, -5, -6, -7, -9, -11, -13, GM-CSF, G-CSF, leukaemia inhibitory factor, oncostatin-M, ciliary neurotrophic factor, thrombopoieitin, calcitonin and leptin) can also be modulated to achieve the desired effect. Thus, modulators of CMT activity are useful in modulating cell proliferation, signal transduction, neuroregeneration and neurodegneration, angiogenesis, apoptosis, immunity inflammation, cell trafficking, protein synthesis and transport, and parasite-host interactions.

CMT modulators (activators or inhibitors) may be formulated according to conventional methodology, depending on the exact nature of the modulator, and will typically comprise the modulator or a precursor thereof in association with a biologically acceptable carrier. In considering various therapies, it is understood that such therapies may be targeted to tissues demonstrated to express CMT.

Delivery of the modulator to the affected cells and tissues can be accomplished using appropriate packaging or administration systems. For example, the modulator may be formulated for therapeutic use with agents acceptable for pharmaceutical administration and delivered to the subject by acceptable routes to produce a desired physiological effect. An effective amount is that amount that produces the desired physiological effect.

### EXAMPLES

The examples are described for the purposes of illustration and are not intended to limit the scope of the invention. Variations in the design and detection of the assays will be apparent to one of ordinary skill in the art.

Methods of molecular genetics, protein and peptide biochemistry and immunology referred to but not explicitly described in this disclosure and examples are reported in the scientific literature and are well known to those skilled in the art. For example, standard methods in genetic engineering are carried out essentially as described in Sambrook et al., Molecular Cloning: A laboratory manual, 2nd Ed., Cold Spring Harbor Laboratory Press, Cold Spring Harbor NY, 1989.

### Example 1: Cell-based assays for CMT activity

The present inventors have designed, constructed and characterized a cell-based system that allows for efficient assaying of CMT activity. The system is exemplified here using a surface-associated marker protein containing a C-mannosylation motif that, when modified, can be recognized by a specific antibody.

### cDNA constructs

### Soluble form of GST-AWAQWA

PCR was used to generate a construct encoding the secreted form of glutathione S-transferase (GST, from Schistosoma japonicum) containing a thrombin cleavage site and AWAKWA, a substrate for the C-mannosyltransferase (CMT), at the C-terminal end. A forward primer, 5'-ACAGAAGCTTCCACCAATG**GTTCC AAAACTGTTCACTTCCCAAATTTGTCTGCTTCTTCTGTTGGGGCTTCTGGCT GTGGAGGGC**TCACTCCATGTCTCCCCTATACTAGGTTATTG3' (SEQ ID NO:2), carrying the signal sequence from eosinophil derived neurotoxin (EDN; shown in bold) (Newton, D.L. et al., 1994, J. Biol. Chem., 269: 26739-26745), annealed to the N-terminal end of GST in pGEX-2T (Pharmacia) and a reverse primer 5'-ATATGAATTCTCAGTCAGTCAAGCCCATTTAGCCCAAGCGGATCCA CGCGGAACC-3' (SEQ ID NO:3) encoding the CMT substrate site AWAKWA,(SEQ ID NO:1) annealed to the thrombin sequence at the C-terminal end of GST in pGEX-2T. The PCR product was subcloned into the Hind III and EcoR I sites of pSMCi (Asselbergs, F.A. & Grand, P., 1993, Anal. Biochem., 209: 327-331) to yield pSMCi-ssEDN-GST-AWAKWA (SEQ ID NO:1). A number of different CMT substrates were generated by site-directed mutagenesis using degenerate primers. One of these constructs encoded the protein GST-AWAQWA (SEQ ID NO:1), which was found to be efficiently C-mannosylated (90%) by CMT following expression in HEK-293 cells and was recognised by the modification specific antibody α5-10 (Krieg, J. et al., 1997, J. Biol. Chem., 272: 26687-26692). This construct was used as a template for generating pSMCi-APN-GST-AWAQWA (SEQ ID NO:1), encoding the membrane bound form of this CMT substrate.

### Cell surface bound APN-GST-AWAQWA

A cell surface form of GST-AWAQWA (SEQ ID NO:1) was made by ligating the cDNA encoding the N-terminal membrane-anchor domain of human aminopeptidase N (APN) to the cDNA encoding GST-AWAQWA (SEQ ID NO:1) via an Spe I restriction site and subcloning into the mammalian expression vector pSMCi using the Hind III/ EcoR I restriction sites.

The N-terminal portion of the fusion protein comprising the cytoplasmic-, the transmembrane- and the stalk region of human aminopeptidase N (APN, 204 bp) was cloned out by PCR from the template pTEJ4-humanAPN (Vogel, L.K. et al., 1992, J. Biol. Chem., 267: 2794-2797) using the forward primer 5'-GGGGTACCAGATCTAAGCTT**GCCACCATGG**CCAAGGGCTTCTATATTTCC-3' (SEQ ID NO:4) and the reverse primer 5'-GACTAGT**ACCCTGAAAATACAAAT TCTC**ACTTTGGTCCAAGGTGGTGGCC-3' (SEQ ID NO:5). The forward primer includes a Hind III restriction site (underlined) and a Kozak sequence (in bold). A tobacco etch virus (TEV) protease site (21 bp, in bold) was introduced via the reverse primer followed by an Spe I restriction site (underlined).

The C-terminal part of the fusion protein included GST followed by a thrombin cleavage site and the CMT substrate site AWAQWA (SEQ ID NO:1). A 699 bp fragment from pSMCi -ssEDN-GST-AWAQWA cDNA was obtained by PCR using the forward primer 5'-GACTAGTTCCCCTATACTAGGTTATTGG-3' (SEQ ID NO:6), with the Spe I restriction site (underlined) and the reverse primer 5'-GCTCTAGAGAATTCCTATCAAGCC CACTGAGCCCAAGCGGAT-3' (SEQ ID NO:7). The reverse primer included the AWAQWA substrate, stop signals and an EcoR I restriction site (underlined).

PCR products were gel purified using QiaExII (Qiagen) and ligated into the pSMCi mammalian expression vector via the Hind III/ EcoR I restriction sites. Following transformation, clones were selected by cleavage with restriction endonucleases and the insert was fully sequenced in both directions. This construct is referred to as pSMCi-APN-GST-AWAQWA (SEQ ID NO:1).

The insert from pSMCi-APN-GST-AWAQWA (SEQ ID NO:1) was excised and ligated into pcDNA3.1 via the Hind III/ EcoR I restriction sites. The resulting construct, pcDNA3.1-APN-GST-AWAQWA (SEQ ID NO:1), was used to establish stable cell lines by selecting transfectants in the presence of neomycin. The cDNA sequence of the pSMCi-APN-GST-AWAQWA (SEQ ID NO:1) insert (924 bp) is provided below:
ATGGCCAAGGGCTTCTATATTTCCAAGTCCCTGGGCATCCTGGGGATCCTC CTGGGCGTGGCAGCCGTGTGCACAATCATCGCACTGTCAGTGGTGTACTCC CAGGAGAAGAACAAGAACGCCAACAGCTCCCCCGTGGCCTCCACCACCCC GTCCGCCTCAGCCACCACCAACCCCGCCTCGGCCACCACCTTGGACCAAA GTGAGAATTTGTATTTTCAGGGTACTAGTTCCCCTATACTAGGTTATTGGAAA ATTAAGGGCCTTGTGCAACCCACTCGACTTCTTTTGGAATATCTTGAAGAAA AATATGAAGAGCATTTGTATGAGCGCGATGAAGGTGATAAATGGCGAAACA AAAAGTTTGAATTGGGTTTGGAGTTTCCCAATCTTCCTTATTATATTGATGGT GATGTTAAATTAACACAGTCTATGGCCATCATACGTTATATAGCTGACAAGC ACAACATGTTGGGTGGTTGTCCAAAAGAGCGTGCAGAGATTTCAATGCTTG AAGGAGCGGTTTTGGATATTAGATACGGTGTTTCGAGAATTGCATATAGTAA AGACTTTGAAACTCTCAAAGTTGATTTTCTTAGCAAGCTACCTGAAATGCTGA AAATGTTCGAAGATCGTTTATGTCATAAAACATATTTAAATGGTGATCATGTA ACCCATCCTGACTTCATGTTGTATGACGCTCTTGATGTTGTTTTATACATGGA CCCAATGTGCCTGGATGCGTTCCCAAAATTAGTTTGTTTTAAAAAACGTATTG AAGCTATCCCACAAATTGATAAGTACTTGAAATCCAGCAAGTATATAGCATG GCCTTTGCAGGGCTGGCAAGCCACGTTTGGTGGTGGCGACCATCCTCCAAA ATCGGATCTGGTTCCGCGTGGATCCGCTTGGGCTCAGTGGGCT (SEQ ID NO:8)

The translated sequence encoded by the cDNA (308 aa) is:
MAKGFYISKSLGILGILLGVAAVCTIIALSVVYSQEKNKNANSSPVASTTPSASAT TNPASATTLDQSENLYFQGTSSPILGYWKIKGLVQPTRLLLEYLEEKYEEHLYER DEGDKWRNKKFELGLEFPNLPYYIDGDVKLTQSMAIIRYIADKHNMLGGCPKER AEISMLEGAVLDIRYGVSRIAYSKDFETLKVDFLSKLPEMLKMFEDRLCHKTYLN GDHVTHPDFMLYDALDVVLYMDPMCLDAFPKLVCFKKRIEAIPQIDKYLKSSKYI AWPLQGWQATFGGGDHPPKSDLVPRGSAWAQWA (SEQ ID NO:9)

Within the translated sequence, KSLGILGILLGVAAVCTIIALSVVYSQ (amino acids 9-35 of SEQ ID NO:9) is the transmembrane region of APN; EKNKNANSSPVASTTPSASATTNPASATTLDQS (amino acids 36-68 of SEQ ID NO:9) is the stalk domain of APN;
ENLYFQG (amino acids 69-75 of SEQ ID NO:9) is the TEV cleavage site;
LVPRGS (amino acids 297-302 of SEQ ID NO: 9) is the thrombin cleavage site; and
AWAQWA (aminoacids 303-308 of SEQ ID NO: 9) is the CMT substrate.

### Cell culture and transfection

Human embryonic kidney cells (HEK-293T) and baby hamster kidney cells (Py-BHK) were maintained in DMEM supplemented with 10% FCS and transfected with pSMCi-APN-GST-AWAQWA (SEQ ID NO:1) cDNA using Lipofectamine (Invitrogen). The day before transfection, cells were seeded at 2.5 × 10⁶ cells per 100 mm dish. The DNA-Lipofectamine mix was prepared according to the manufacturer's instructions. For each dish, 10 µg of plasmid DNA and 50 µl of Lipofectamine were added in 8 ml of Opti-MEM1 (Invitrogen). After 7 h, an equal volume of DMEM supplemented with 20% FCS was added and incubated overnight. The next morning, transfected cells were incubated with fresh medium and incubated for a further 48 h before harvesting in PBS-0.05% EDTA. The cells were washed with PBS and either analyzed by flow cytometry or digested with TEV protease to recover the GST-fusion protein. Stable cell lines expressing APN-GST-AWAQWA (SEQ ID NO:1) and CD59 on their cell surface were generated by co-transfecting cells with pcDNA3.1-APN-GST-AWAQWA (SEQ ID NO:1) and pcDNA3.1/HygroCD59 and selecting clones in medium containing neomycin and hygromycin.

### Flow cytometry

Cells were labelled with either rabbit anti-GST (Sigma) to detect cell surface protein expression or rabbit α5-10 antibody to identify the mannosylated CMT substrate. We have previously demonstrated that the α5-10 antibody is specific for the mannosylated forms of the CMT substrate and does not recognize the unmodified protein (Krieg, J. et al., 1997). Cells were filtered through a 40 µm strainer (Falcon), counted and resuspended at 10⁷ cells/ml in blocking buffer (PBS, 1% BSA, 10 mM NaN₃, 5% normal goat serum). Following a 30 min incubation on ice, 100 µl of cells were incubated with 100 µl of primary antibody diluted to 5 µg/ml in blocking buffer for 30 min at 4°C. After washing the cells in wash buffer (PBS, 1%BSA,10 mM NaN₃), they were incubated with 100 µl of goat anti-rabbit IgG-FITC (Jackson ImmunoResearch) diluted 1:200 in blocking buffer for 30 min at 4°C. The stained cells were washed and resuspended in 500 µl ice-cold PBS and analyzed on a Becton Dickinson FacsCalibur using CellQuest software. Negative controls consisted of transfected cells probed with normal rabbit IgG or untransfected cells incubated with specific antibodies.The anti-GST antibodies showed that approximately 50% of the cells expressed the GST-CMT substrate fusion protein on their surface. The majority of this protein was C-mannosylated as shown by staining with the α5-10 antibodies. The negative controls showed low background staining. Thus, C-mannosylation of a cell-surface expressed CMT substrate has been established for the first time, allowing a simple format for cell-based assays.

### Example 2:Cell-based assays for CMT activity

The system is exemplified here using the surface-associated marker protein containing a C-mannosylation motif that, when modified, can be recognized by protein chemistry analysis. The methodology was essentially as described in Example 1 but protein chemistry analysis replaces or complements the flow cytometry step used above.

### Protein chemistry analysis

The expressed CMT substrates were purified from transfected cells and analyzed by LC-MS for the presence of C-mannosylated peptides essentially as follows. Approximately 5 x 10⁷ cells were transfected with pSMCi-APN-GST-AWAQWA (SEQ ID NO:1) cDNA and harvested as described above. After washing the cells in ice-cold PBS, the cell pellet was resuspended in a final volume of 500 µl TEV buffer (50 mM Tris-HCl, pH 8.0, 0.5 mM EDTA, 5 mM DTT). One hundred units of TEV protease (Invitrogen) was added and mixed gently for 7 hours at 16°C. FACS analysis showed that more than 70% of the GST-AWAQWA protein (SEQ ID NO:1) was released from the cell surface after 5 hours of incubation.

The cells were pelleted and the supernatant further clarified by centrifugation. The supernatant was diluted to 10 ml with DMEM containing 10% FCS and a protease inhibitor cocktail (20 µl of a 500x stock solution containing 5 mg benzamidine, 1 mg pepstatin A, 1 mg leupeptin, 1 mg antipain, 1 mg antichymotrypsin, 20 mg PMSF in 1 ml 40 % DMSO, 60% ethanol) and incubated with glutathione-agarose beads (Amersham Pharmacia) overnight at 4°C on a roller. The sample was transferred to a BioRad column and the beads washed with 3 ml buffer A (PBS containing 5mM EDTA, 2mM DTT), followed by 3 ml buffer B (buffer A with 250 mM NaCl) and a final wash of 3 ml 50 mM Tris-HCl, pH 8.0. The washed beads were then incubated with shaking for 1 hour at 37°C in 200 µl 50 mM Tris-HCl, pH 8.0, 20 mM reduced glutathione, 2mM DTT. The column was placed over a Microcon UltraFree tube and centrifuged at 1000 rpm for 3 min. The filtrate containing the eluted GST fusion protein was further purified by reverse-phase HPLC using a C4 column. The column was equilibriated in solvent A (0.1% trifluoroacetic acid, TFA), washed with 20% solvent B (0.1% TFA, 80% CH3CN) and eluted using a linear gradient of solvent B over 30 minutes. The protein was monitored at 214 nm. The CMT substrate peptide was released from the GST fusion protein by cleavage with thrombin. Dried protein peaks (approximately 10 µg) were dissolved in 5 µl 9 M urea, followed by the addition of 43 µl 50 mM Tris-HCl, pH 8.0 and 2 µl (4 units) of thrombin and incubated for one hour at room temperature. Analysis of the peptides was carried out by LC-MS using a C18 column equilibrated in 0.05% TFA, 2% CH3CN and developed with a linear gradient of 20-80% solvent B (0.045% TFA, 80% CH3CN) over 30 minutes. The HPLC was interfaced with an API300 mass spectrophotometer. Peptides with the masses expected for GSA(Mannose)WAQWA (1038 Da; amino acids 304-308 of SEQ ID NO:9) and GSAWAQWA (876 Da; amino acids 301-308 of SEQ ID NO:9) were observed.

Although this Example illustrates the invention by identifying the masses of a C-mannosylated CMT substrate and unmodified equivalent, it will be apparent to one of skill in the art that other methods can be easily devised for high-through put formats for identifying the presence of the modified CMT substrate released from the cell surface, using detectable labels or antibodies, for example.

### Example 3 Cell-based assay for CMT activity

In this example, instead of using a protease to release the CMT substrate from the cell substrate, a secreted form of the CMT substrate is used. The GST-AWAQWA (SEQ ID NO:1) fusion protein was obtained from the conditioned medium of cells transfected with pSMCi-ssEDN-GST-AWAQWA (SEQ ID NO:1) described in Example 1. The conditioned medium was concentrated 35-fold by ultrafiltration using a Centricon plus-80 tube (Millipore). GST fusion protein purification and subsequent peptide release is performed as described in Example 2.

### Example 4 Cell-based screen for inhibitors of CMT

The assay described in Example 1 can be used for efficient screening of agents as inhibitors of CMT. Inhibitory agents can be detected by the absence or reduction of antibody binding to the cells. In addition, C-mannosylation requires DPM. A second reporter molecule, such as CD59, containing a glycosylphosphatidylinositol (GPI) anchor can optionally be included, serving as a positive marker to exclude inhibitors of the DPM synthesis pathway.

In brief, human embryonic kidney cells (HEK293T) cells that stably express APN-GST-WAQWA and optionally CD59 are seeded into the wells of a 384-well microtiter plate at approximately 20% confluency. A test agent or combinations of test agents can be added to a final concentration of 1 mM from 10 mM stock solutions in DMSO or other suitable concentrations or solvents, as will be apparent to one of ordinary skill in the art. After addition of the agents cells are grown to approximately 90% confluency, washed and stained with α5-10 antibodies that have been fluorescently labeled with APC, and with anti-CD59 antibodies that have been labeled with FITC. Antibody binding is measured with a 2100 Envision apparatus (Perkin Elmer). Agents that result in reduced binding of α5-10 compared to untreated controls but have no effect on the binding of anti-CD59 are potential specific inhibitors of CMT activity, that is, they do not affect the dolichol-phosphate-mannose (DPM) synthesis pathway, which has additional cellular functions.

### Example 5 Cell-based screen for inhibitors of CMT

HEK293T cells that stably express APN-GST-WAQWA and CD59 are treated with TEV protease as described in Example 2. After washing away the protease, cells are seeded into the wells of a 384-well microtiter plate at approximately 50% confluency. Test agents are added to a final concentration of 1 mM from 10 mM stock solutions in DMSO and incubation is continued for 18 and 36h. Antibody binding and verification of inhibitors is assayed as described in Example 4.

## Claims

1. A method of assaying for C-mannosyltransferase (CMT) activity, said method comprising the steps of:
i) providing a cell comprising CMT and a fusion protein, said fusion protein comprising a CMT substrate and a transmembrane domain,
ii) providing conditions conducive to forming a C-mannosylated CMT substrate by action of said CMT on said CMT substrate in said cell; and
iii) detecting said C-mannosylated CMT substrate on the surface of said cell.

2. The method according to claim 1, wherein said C-mannosylation of said substrate is detected using an antibody.

3. The method according to claim 2, wherein said antibody is specific for C-mannosylated CMT substrate.

4. The method according to any one of the preceding claims, wherein said C-mannosylation of said substrate is detected using a label.

5. The method according to claim 1, further comprising cleaving said fusion protein with a protease.

6. A method of identifying an agent effective in modulating C-mannosyltransferase (CMT) activity, said method comprising the steps of the method of any one of the preceding claims in the presence of a putative agent and detecting an increase or decrease in the amount of C-mannosylated CMT substrate.

7. The method according to claim 6, wherein a decrease in the amount of C-mannosylation is achieved and said modulation is inhibition of CMT activity.

8. The method according to claim 6, wherein an increase in the amount of C-mannosylation is achieved and said modulation is activation of CMT activity.

9. The method of any one claims 6 to 8, said method further comprising detecting the presence of a GPI anchor.

## Patentansprüche

1. Verfahren zum Testen auf C-Mannosyltransferase (CMT) Aktivität, wobei das Verfahren die folgenden Schritte umfasst:
i) Bereitstellung einer Zelle, die CMT und ein Fusionsprotein umfasst, wobei das Fusionsprotein ein CMT Substrat und eine Transmembrandomäne umfasst,
ii) Bereitstellung von Bedingungen, die eine Bildung eines C-mannosylierten CMT Substrats durch die Wirkung der CMT auf das CMT Substrat in der Zelle erlauben, und
iii) Detektion des C-mannosylierten CMT Substrats auf der Oberfläche der Zelle.

2. Verfahren nach Anspruch 1, worin die C-Mannosylierung des Substrats mittels eines Antikörpers detektiert wird.

3. Verfahren nach Anspruch 2, worin der Antikörper für ein C-mannosyliertes CMT Substrat spezifisch ist.

4. Verfahren nach einem der vorangehenden Ansprüche, worin die C-Mannosylierung des Substrats mittels einer Markierung detektiert wird.

5. Verfahren nach Anspruch 1, das ferner die Spaltung des Fusionsproteins mit einer Protease umfasst.

6. Verfahren zur Identifizierung eines Mittels, das bei der Modulierung der C-Mannosyltransferase (CMT) Aktivität wirksam ist, wobei das Verfahren die Schritte des Verfahrens nach einem der vorangehenden Ansprüche in Gegenwart eines putativen Mittels und die Detektion einer Zunahme oder Abnahme der Menge an C-mannosyliertem CMT Substrat umfasst.

7. Verfahren nach Anspruch 6, worin eine Abnahme der Menge an C-Mannosylierung erreicht wird und die Modulierung die Hemmung der CMT Aktivität ist.

8. Verfahren nach Anspruch 6, worin eine Erhöhung der Menge an C-Mannosylierung erreicht wird und die Modulierung die Aktivierung der CMT Aktivität ist.

9. Verfahren nach einem der Ansprüche 6 bis 8, wobei das Verfahren ferner die Detektion der Gegenwart eines GPI Ankers umfasst.

## Revendications

1. Procédé de détermination de l'activité C-mannosyltransférase (CMT), ledit procédé comprenant les étapes consistant à :
i) fournir une cellule comprenant une CMT et une protéine de fusion, ladite protéine de fusion comprenant un substrat de CMT et un domaine transmembranaire ;
ii) fournir les conditions conduisant à former un substrat de CMT C-mannosylé par l'action de ladite CMT sur ledit substrat de CMT dans ladite cellule ; et
iii) détecter ledit substrat de CMT C-mannosylé à la surface de ladite cellule.

2. Procédé selon la revendication 1, dans lequel ladite C-mannosylation dudit substrat est détectée en utilisant un anticorps.

3. Procédé selon la revendication 2, dans lequel ledit anticorps est spécifique du substrat de CMT C-mannosylé.

4. Procédé selon l'une quelconque des revendications précédentes, dans lequel ladite C-mannosylation dudit substrat est détectée en utilisant un marqueur.

5. Procédé selon la revendication 1, comprenant en outre le clivage de ladite protéine de fusion avec une protéase.

6. Procédé d'identification d'un agent efficace pour moduler l'activité C-mannosyltransférase (CMT), ledit procédé comprenant les étapes du procédé selon l'une quelconque des revendications précédentes, en présence d'un agent putatif et la détection d'une augmentation ou d'une réduction de la quantité de substrat de CMT C-mannosylé.

7. Procédé selon la revendication 6, dans lequel on obtient la réduction de la quantité de C-mannosylation, et ladite modulation est une inhibition de l'activité CMT.

8. Procédé selon la revendication 6, dans lequel on obtient une augmentation de la quantité de C-mannosylation, et ladite modulation est une activation de l'activité CMT.

9. Procédé selon l'une quelconque des revendications 6 à 8, ledit procédé comprenant en outre une détection de la présence d'un dispositif d'ancrage de GPI.
